# NEUE EUROPÄISCHE PATENTSCHRIFT

(11) **EP 0 481 226 B2**
(45) Veröffentlichungstag und Bekanntmachung des Hinweises auf die Entscheidung über den Einspruch: **23.04.2003**
(45) Hinweis auf die Patenterteilung: 08.06.1994
(21) Anmeldenummer: 91115709.7
(22) Anmeldetag: 17.09.1991
(51) Int. Cl.: B01J 20/26, A61L 15/00

(54) **Verfahren zur Herstellung von Wasserabsorptionsmaterial auf Polymerbasis und dessen Verwendung**
Process for manufacturing water absorption material based on polymer and its use
Procédé pour la fabrication de matériau d'absorption d'eau à base de polymère et son utilisation

(30) Priorität: 19.09.1990 DE 4029591
(43) Veröffentlichungstag der Anmeldung: 22.04.1992
(73) Patentinhaber: Chemische Fabrik Stockhausen GmbH, 47805 Krefeld (DE)
(72) Erfinder: Chmelir, Miroslav, Dr., W-4150 Krefeld (DE)
(74) Vertreter: Kahlhöfer, Hermann, Dipl.-Phys.

(56) Entgegenhaltungen:
- EP-A- 0 071 063
- EP-A- 0 238 050
- EP-A- 0 303 440
- EP-A- 0 376 118
- WO-A-87/03208
- US-A- 4 116 899

## Beschreibung

Die Erfindung betrifft ein Verfahren zur Herstellung von Absorptionsmaterialien, die aus einer Kombination von synthetischen Polymeren und anderen Substanzen, bevorzugt von gut oder begrenzt wasserlöslichen Substanzen, die in den Polymerkörper eingebunden sind, bestehen. Ein solches Absorptionsmaterial, das Wasser und wäßrige Flüssigkeiten schnell aufnimmt, kann dann zur Aufnahme und/oder Zurückhaltung von Wasser und/oder wäßrigen Lösungen und zur nachfolgenden gesteuerten Abgabe von Wasser und der im aufgequollenen Polymergel enthaltenen und in wäßrigem Medium gelösten Substanzen (Komponente B) an andere Körper, wie z.B. in Nährböden für verschiedene Kulturen, beim Dosieren von Nährstoffen oder Arzneimitteln oder als wasser- und düngemittelspeicherndes Bodenverbesserungsmittel verwendet werden.

In den letzten Jahren wurde eine Anzahl verschiedener Polymerisate entwickelt, die hohes Absorptionsvermögen für Wasser und Körperflüssigkeiten aufweisen. Zu den vollsynthetischen, in zahlreichen Patenten beschriebenen Absorptionsmitteln gehören vernetzte Polymere und Copolymere auf Acryl- oder Methacrylsäurebasis (DE-PS 244 236; DE-OS 26 14 662; DE-OS 26 53 135; DE-OS 26 50 377; DE-OS 28 13 634) oder Acrylamidopropansulfonsäurecopolymerisate (DE-PS 31 24 008).

Weitere Produkte wurden auf Stärkebasis, wie z.B. Stärke-Acrylnitril-Pfropfpolymerisate nach US-PS 3,997,484; US-PS 4,155,888 oder Cellulosebasis, Carboxymethylcellulose nach GB-PS 1 159 949 und auf Polysaccharidbasis nach DE-OS 26 50 377 hergestellt.

Die bekannten synthetischen Absorptionsmittel sind vernetzte Polymerisate, die praktisch wasserunlöslich sind und im Gleichgewicht das Vielfache ihres Gewichts an Wasser oder anderen wäßrigen Lösungen absorbieren können. Solche Produkte werden vorwiegend in der Hygieneindustrie (Windeln, Damenbinden), aber auch in der Pflanzenzucht als wasserspeichernde Bodenverbesserungsmittel eingesetzt, wie z.B. in der DE-PS 27 37 941, oder vermischt mit Kaolin, Fuller-Erden, Talk, Bentonit oder Aluminiumsilikat in der EP-PS 0 072 213 beschrieben.

Abmischungen von synthetischen Absorptionsmitteln mit den meistens gut oder mindestens begrenzt wasserlöslichen Düngemitteln sind bereits bekannt, aber die Einarbeitung des Düngers in das Absorptionsmittel ist schwierig. Nach der EP-PS 0 181 983 wird z.B. das Düngemittel während der Polymerisation zu dem Acrylamidhomo- oder -copolymerisat in einer Menge von 10 bis 50 Gew% zugesetzt. Gemäß EP 0 186 721 wird der Dünger zu einem sehr verdünnten Polyacrylamidgel (2 Gew%) zugegeben, und in der JP 60-141192 (CA 103 (25):214126g) wird ein pulverförmiges Acrylamid/Acrylsäure-Copolymerisat in Düngemittellösung getaucht und danach getrocknet. Eine komplizierte Mischung von Düngemittel mit Naturgummi wie Guargummi oder Johannisbrotkernmehl in Anwesenheit von hochmolekularen Polycarboxylsäuren und Borsäure bzw. Borsalzen ist in der US-PS 4,052,190 beschrieben.

Der Zusatz eines Düngemittels während der Polymerisation zur Monomerlösung beeinflußt wegen der im Düngemittel enthaltenen Verunreinigungen (Schwermetalle) negativ den Polymerisationsverlauf, die Polymerisation verläuft nicht vollständig, und es resultiert daher ein Polymerisat mit sehr hohem Restmonomergehalt. Weitere erwähnte Verfahren, die diese Abmischung in sehr hoher Verdünnung durchführen, sind kaum wirtschaftlich, denn zur Herstellung eines pulverförmigen Produktes müssen hohe Wassermengen wieder durch Trocknung entfernt werden.

Für viele Anwendungen von Absorptionsmaterial ist es wünschenswert, noch andere meistens gut oder begrenzt lösliche Verbindungen in das Absorptionsmittel einzubauen, um dann in gequollenem Zustand eine gesteuerte und bevorzugt verzögerte Abgabe dieser Substanzen an andere Körper (z.B. Düngemittel an die Pflanzen) zu erreichen.

Die einfachste Lösung dieser Aufgabe, eine derartige Verbindung (im weiteren als Komponente B bezeichnet) direkt zur Monomerlösung der Komponente A (synthetisches Polymerisat) zuzusetzen, zu homogenisieren und danach die Polymerisation zu starten, wie in den EP-PS 0 181 983, EP-PS 071 063 und DE-PS 31 28 100 beschrieben, ist nur dann durchführbar, wenn die Komponente B den Polymerisationsverlauf nicht negativ beeinflußt.

In den übrigen Fällen beeinflußt die Komponente B mehr oder weniger den Polymerisationsverlauf durch die Störung der Start- oder Wachstumsreaktion (Reaktion mit den Primärradikalen, Übertragung, Termination). Auch kleine Mengen an Verunreinigungen in der sonst für die Polymerisation unschädlichen Komponente B können die Polymerisation stark beeinflussen, wie z.B. Spurenmengen an Schwermetallen in den üblichen Düngemitteln, so daß eine gleichmäßige, bis zum vollständigen Umsatz geführte Polymerisation unmöglich ist. Besonders die Versuche, größere Mengen an Komponente B in das Polymerisat einzuarbeiten, führen zu einer unvollständigen Polymerisation und zu einem Endprodukt mit recht hohem Restmonomergehalt.

Die Aufgabe der Erfindung ist daher, in die bekannten als Absorptionsmittel eingesetzten synthetischen Polymerisate mit hohem Aufnahmevermögen für Wasser und wässrige Flüssigkeiten, die gut oder begrenzt wasserlösliche Substanzen in beliebigen, vorzugsweise größeren Mengen einzuarbeiten, um Endprodukte mit verzögerter Abgabe der wasserlöslichen Substanzen an andere Körper unter Beibehaltung der guten Absorptionseigenschaften zu erhalten.

Diese Aufgabe wird erfindungsgemäß durch ein Verfahren nach Anspruch 1 und einem Absorptionsmaterial nach Anspruch 11 gelöst. In dem Verfahren wird die Komponente B in Pulverform oder in gelöster bzw. flüssiger Form in der Endphase des Herstellungsprozesses des synthetischen, vernetzten Polymerisats A, d.h. in der Endphase der Polymerisation in wäßriger Lösung, zugesetzt und unter Bildung eines wirksamen Absorptionsmaterials in das synthetische Polymerisat durch anschließende Trocknung, falls notwendig, eingebunden.

Die Herstellung der Komponente A erfolgt nach dabei bekannten Methoden. So kann die Herstellung der Komponente A als gequollenes Polymergel diskontinuierlich in einem Polymerisationsgefäß oder kontinuierlich auf einem endlosen band erfolgen. Gemäß DE-PS 35 44 770 wird z.B. die Polymerisation in einer wäßrigen Lösung, enthaltend das wasserlösliche Monomere und ggf. die Comonomeren, in einer Konzentration von 2,2 bis 8,3 Mole polymerisationsfähiger Doppelbindungen pro Kilo Monomerenlösung, insbesondere 3,5 bis 6,25 Mole (entsprechend 16 - 60 Gew%, insbesondere 25 - 45 Gew% Acrylsäure, wenn diese als Monomeres verwendet wird) und im Temperaturbereich von etwa 10 - 120°C durchgeführt.

Als Komponente A kommen in Frage in erster Linie die Polymerisate von Acrylsäure und Methacrylsäure, allein als Homopolymerisat oder als Copolymerisat, ferner aber auch die Polymerisate von anderen waserlöslichen Monomeren wie Acrylamid sowie polymerisationsfähige Säuren und ihre Salze, insbesondere Malein-, Fumar-, Itacon-, Vinylsulfon- oder 2-Acrylamido-2-methylpropansulfonsäure. Ferner können hydroxygruppenhaltige Ester polymerisationsfähiger Säuren, insbesondere die Hydroxyethyl- und Hydroxypropylester der Acryl und der Methacrylsäure, verwendet werden, ebenso aminogruppenhaltige und ammoniumgruppenhaltige Ester und Amide polymerisationsfähiger Säuren, wie die Dialkylaminoester, insbesondere die Dimethylund die Diethylaminoalkylester der Acryl- und der Methacrylsäure, sowie die Trimethyl- und die Triethylammoniumalkylester und die entsprechenden Amide. Weiterhin werden in geringen Anteilen vernetzende Monomere, wie z.B. Monomere mit mehr als einer polymerisationsfähigen Gruppe im Molekül, zusammen mit den vorstehenden Monomeren polymerisiert.

Die vorstehenden Monomeren können allein zu vernetzten Homo- oder untereinander zu vernetzten Copolymerisaten polymerisiert werden.

In geringen Mengen können noch in Wasser schwerlösliche oder sogar wasserunlösliche Monomere wie (Meth)acrylnitril, Vinylpyridin und Vinylacetat copolymerisiert werden wie die Ester der Acryl- und/oder Methacrylsäure mit C₁-C₁₀-Alkoholen, Styrol und alkylierte Styrole. Im allgemeinen liegt der Anteil an den wasserlöslichen Monomeren bei 40 - 100 Gew%, bezogen auf die Gesamtheit der Monomeren. Der Anteil an den vernetzenden Monomeren liegt bei 0 - 20 Gew%, vorzugsweise bei 0,01 - 2,0 Gew%, bezogen auf die Gesamtheit der Monomeren. Die wasserunlöslichen, hydrophoben Monomeren machen in der Regel 0-40 Gew% der Monomeren aus.

Als vernetzende Monomere seien bi- oder mehrfunktionelle Monomere, z.B. Amide, wie das Methylenbisacryl- bzw. -methacrylamid oder Ethylenbisacrylamid, ferner Ester der ungesättigten Mono- oder Polycarbonsäuren von Polyolen, wie Diacrylate oder Triacrylate, z.B. Butandiol- oder Ethylenglykoldiacrylat bzw. -methacrylat, Trimethylolpropantriacrylat, ferner Vinylmethacrylat und Allylverbindungen wie Allyl(meth)acrylat, Triallylcyanurat, Maleinsäurediallylester, Polyallylester, Tetraallyloxiethan, Triallylamin, Tetraallylethylendiamin, Allylester der Phosphorsäure bzw. phosphorigen Säure, ferner vernetzungsfähige Monomere, wie die N-Methylolverbindungen von Amiden, wie Methacrylamid bzw. Acrylamid und die davon abgeleiteten Ether.

Die Polymerisation kann durch chemische Katalyse und/oder durch energiereiche Strahlung/Licht initiiert werden. Als geeignete Katalysatoren können z.B. Peroxiverbindungen wie Kaliumperoxidisulfat, Wasserstoffperoxid, organische Peroxide wie Benzoylperoxid, tert. Butylhydroperoxid, tert. Butylperpivalat, Redox-Systeme, wie z.B. Kaliumperoxidisulfat-Natriumdisulfit, Wasserstoffperoxid-Hydroxylaminchlorid oder Azoinitiatoren wie AIBN (2,2'-Azobis-(isobutyronitril)) oder 2,2'-Azobis(2-amidinopropan)dihydrochlorid verwendet werden. Als Photoinitiatoren können z.B. Benzoin und seine Derivate, z.B. Benzoinetherwie Benzoin-Ethyl-Propyl-Ether, Benzil und seine Derivate, wie Benzilketale oder Acryldiazoniumsalze, Acetophenonderivate und andere allein oder in Gemischen verwendet werden. Im allgemeinen liegt der Gehalt an Photoinitiatoren bei 0,002 bis 2,0 Gew%, vorzugsweise 0,01 bis 0,2 Gew%, bezogen auf die eingesetzten Monomeren. Der Gehalt an Katalysatoren liegt im allgemeinen bei 0,02 bis 5,0 Gew%, vorzugsweise zwischen 0,20 und 2,0 Gew%, bezogen auf die Monomeren.

Als Komponente B können anorganische oder organische Verbindungen, die bei normaler Temperatur als rieselfähiges Pulver vorliegen und gut oder mindestens begrenzt wasserlöslich sind, verwendet werden. Solche Verbindungen sind z.B. Salze der anorganischen oder organischen Säuren, bevorzugt die Ammonium-, Natrium-, Kalium-, Lithium-, Calcium-, Magnesium-, Zink-, Aluminium- oder Eisensalze. Als Salze anorganischer Säuren werden bevorzugt die Chloride, Bromide, Jodide, Sulfate, Hydrosulfate, Phosphate, Hydrogen- oder Dihydrogenphosphate, Tetraborate, Nitrate, Carbonate oder Hydrogencarbonate, als Salze organischer Carbonsäuren die Salze der Essig-, Ameisen-, Adipin-, Citronen- oder Weinsäure oder auch die Salze von niedermolekularen polymeren Carbon- bzw. Sulfonsäuren mit Mol-Gewichten zwischen 100 und 1000000, vorzugsweise 2000 bis 200000, auf der Basis von Homo- oder Copolymerisaten ungesättigter Mono- oder Dicarbonsäuren, Sulfonsäuren, Aldehyden, Alkoholen sowie (Meth-)Acrylamid. Es können auch die anorganischen oder organischen Säuren selbst, sofern sie wasermischbar sind, als Komponente B verwendet werden. Geeignete anorganische Säuren sind Borsäure oder Phosphorsäure. Geeignete organische Säuren sind Monocarbonsäuren oder Polycarbonsäuren wie Citronen-, Wein- oderAdipinsäure oder niedermolekulare polymere Carbon- bzw. Sulfonsäuren mit Molekulargewichten zwischen 200 und 1000000 g/Mol, vorzugsweise zwischen 2000 und 200000 g/Mol auf der Basis von Homo- oder Copolymerisaten ungesättiger Mono- oder Dicarbonsäuren, Sulfonsäuren, Aldehyden, Alkoholen sowie (Meth-)Acrylamid.

Besonders bevorzugt als Komponente B werden die Salze der anorganischen oder organischen Säuren sowie die Säuren selbst, wie Ammonium-, Natrium- oder Kaliumnitrate, -phosphate, -sulfate oder Phosphorsäure, die als Düngemittel geeignet sind.

Weiter sind geeignet: die wasserlöslichen, festen Derivate und Carbonsäure wie Aminocarbonsäuren, Amide oder Diamide, vorzugsweise Acetamid, Harnstoff und Harnstoff-Derivate wie Thioharnstoff, Methyl- oder Ethylharnstoff.

Zusätzlich können zu der Komponente B noch natürliche Polymerisate auf Polysaccharidbasis, wie modifizierte Cellulose und Cellulosederivate, z.B. Alkyl-, Hydroxyalkyl-, Carboxymethylcellulose, Gummizarze, z.B. Guar-, Johannisbrotkernmehl, Traganthgummi, Gummi Arabicum, Pektin u.a., Stärke und Stärkederivate, wie z.B. Mais-, Korn-, Kartoffelstärke, Amylose, Amylopektin, Dextrin, Dextran, modifizierte Stärke, Hydroxyethylstärke, kationische Stärke, Stärkepfropfpolymerisate u.a. zugesetzt werden.

Neben diesen Naturpolymeren können zu der Komponente B noch andere Materialien mit großer Oberfläche, wie z.B. Fasermaterial aus Naturfasern, bevorzugt Baumwolle-, Hanf-, Wolle- und Seidenfasern, weiterhin Fasermaterial aus Cellulosefasern wie Viskose-, Acetat- und Triacetatfasern oder aus synthetischen Fasern auf der Basis von Polyester, Polyolefinen, Polyacrylnitril, Polyamid, Polyvinylalkohol, -acetat und -chlorid, Polyurethan, Polyvinylharnstoff und der Copolymerisate von diesen Polymeren, verwendet werden. Die Fasermaterialien können bevorzugt als Kurzschnittfaser von einer Länge von 0,1 bis 60 mm in die Komponente gemeinsam mit der Komponente B eingearbeitet werden.

Der Zusatz von Natur- oder synthetischen Fasern zu dem quellfähigen, B-Komponente enthaltenden Polymerisat bewirkt eine schnellere Aufnahme von Wasser, wäßrigen Flüssigkeiten und den in Wasser gelösten Stoffen (Düngemittellösung) durch das quellfähige Polymerisat. Diese erhöhte und vor allem schnellere Aufnahme der Flüssigkeit ist auf die Kapillarwirkung des Faseranteils zurückzuführen. Es ist daher auch möglich, durch den Faserzusatz (und die Fasermenge) zu dem B-Komponente enthaltenden Polymerisat die Geschwindigkeit der Abgabe des wasserlöslichen Düngemittels an das überschüssige Wasser (Regen, Gießen) und sein Weiterführen an andere Körper (z.B. Pflanzen) sowie die Aufnahme der Düngemittellösung durch ein teilgequollenes oder halbtrockenes Polymerisat in bestimmten Grenzen noch zusätzlich zu steuern.

Weitere Materialien wie Riechstoffe zur Parfümierung des Endproduktes, Desinfektionsmittel, antibakterielle Mittel, Farbstoffe, aber auch neutrale Füllstoffe zur Streckung der Komponente A, wie z.B. Holzmehl, Torf, gemahlene Walnußschalen, Kernobstschalen, chitinhaltiges Mehl, Sand, Erde für Pflanzenzucht oder andere Streckmittel, können auch als Zusatz verwendet werden. Letztlich kann auch die feingemahlene Komponente A getrocknet in Pulverform oder in teilgequollener Form mit der Komponente B zum Polymergel der Komponente A zugesetzt werden.

Das erfindungsgemäße Verfahren besteht darin, daß die Komponente B als Pulver oder in flüssigem Zustand bzw. als eine Lösung in der Endphase der Herstellung der Komponente A, d.h. erst nachdem ein Umsatz von mindestens 90 %, bevorzugt mindestens 95 % und besonders bevorzugt mehr als 98 %, erreicht wird, zu dem gequollenen Polymergel der Komponente A zugesetzt und danach weiter verarbeitet wird.

Durch weitere Verarbeitung der Abmischung der beiden Komponenten in einem Mischer mit rotierendem Mischwerkzeug und eventueller nachträglicher Trocknung der Polymergelmasse bei einer Temperatur in einem Bereich von 50 bis 260°C erhält man ein Endprodukt, in dem die Komponente B (z.B. ein Düngemittel) im synthetischen Polymerisat so eingebunden ist, daß die in Wasser lösliche Komponente B aus dem gequollenen Endprodukt deutlich langsamer extrahierbar ist als bei einem physikalischen Gemisch der beiden Komponenten.

Eine verlangsamte Extraktion des Düngemittels aus dem Polymerkörper, d.h. eine verlangsamte Abgabe des Düngemittels an das überschüssige Wasser, kann sich sehr positiv in der praktischen Anwendung in zweierlei Hinsicht zeigen: Erstens bleibt das Düngemittel bei großen Regenfällen längere Zeit in dem Polymerkörper gebunden und steht daher auch den Pflanzen für längere Zeit zur Verfügung und zweitens werden beim Regen oder beim Gießen die Düngemittelbestandteile nicht so schnell ausgewaschen, wodurch eine nicht so starke Belastung des Grundwassers erreicht wird. In beiden Fällen erzielt man eine bessere Ausnutzung des Düngemittels für das Pflanzenwachstum.

Die Einarbeitung der Materialien mit großer Oberfläche wie z.B. Kurzschnittfaser zusammen mit der Komponente B in das Polymergel bringt zusätzliche Vorteile bei der Trocknung des Endproduktes. Durch die vergrößerte Oberfläche werden die Trocknungszeiten kürzer, so daß die Trocknung auch bei niedrigerer Temperatur effektiv erfolgen kann.

Die Abmischung der beiden Komponenten kann in einer geeigneten Mischeinrichtung erfolgen. Der in den nachfolgenden Beispielen benutzte Mischer bestand aus einem vertikal oder horizontal gelegenen Metallzylinder, dessen Mischwerkzeug mit Leitschaufeln, die die Wände des Mischerzylinders gründlich abstreifen, ausgerüstet war. Nachdem eine bestimmte Drehzahl des Rührwerks erreicht wurde, erfolgte auf der ganzen Länge des Metallzylinders eine gleichmäßige Durchmischung und an den Wänden eine Durchknetung der beiden Komponenten.

Für eine gleichmäßige Durchmischung der beiden Komponenten A und B kann auch ein Trogkneter mit Doppel-U-Querschnitt verwendet werden. Im Trog bewegt sich ein Knetwellenpaar gleich- oder gegensinnig, gleich oder verschieden schnell, wobei auch die Schaufelform je nach Mischgut gezielt gewählt werden kann.

Zum Beispiel kann zu diesem Zweck ein Trogkneter der Firma Werner & Pfleiderer, Stuttgart, mit gezähnten, gegensinnig laufenden Sigma-Schaufeln benutztwerden. Zum kontinuierlichen Betrieb kann der Trogkneter mit einem Austragzylinder und einer Austragschnecke, die entweder gegenläufig zum Troginnern arbeitet und damit den Misch- und Knetvorgang verstärkt oder die in umgekehrter Richtung zur Austragung des Mischgutes geschaltet wird, ausgestattet werden.

Zu den weiteren, für einen kontinuierlichen Betrieb geeigneten Maschineneinrichtungen gehören: Einwellenmischer wie Einschneckenextruder, Ko-Kneter, Zweiwellenmischer mit gleichläufiger oder gegenläufiger Doppelschnecke, konische Cotruder-Schnecke, Zweiwellendurchlaufkneter und kontinuierliche Mehrwellengeräte, wie z.B. ein Vierschneckenextruder.

Als Bindemittel für die beiden Komponenten A und B kann man noch zusätzlich als Hilfsmittel zur Bindung der beiden Komponenten die nieder- oder hochmolekularen, wasserlöslichen oder wasserquellbaren Polymerisate auf synthetischer oder natürlicher Grundlage (z.B. Polysaccharide in gelöstem oder aufgequollenem Zustand) verwenden. Beispiele für wasserlösliche oder wasserquellbare Polymerisate auf synthetischer Basis sind die Polymeren oder Copolymeren auf der Basis von (Meth-)Acrylsäure oder (Meth-)Acrylsäurederivaten wie die Homo- oder Copolymere der Acryl-, Methacryl-, 2-Acrylamido-2-methylpropansulfonsäure, der Salze dieser Säuren, des Acryl- oder Methacrylamids untereinander oder mit Vinylpyrrolidon und/oder Vinylacetat sowie Polyvinylalkohol.

Es können aber auch nieder- oder hochmolekulare Polymerisate, die als Emulsionspolymerisate in einer wäßrigen Dispersion in Form der durch Emulgator solubilisierten winzigen kugelförmigen Partikel vorhanden sind, verwendetwerden, wobei beide Emulsionsformen "Öl-in-Wasser" (für wasser unlösliche Polymere) sowie "Wasser-in-Öl" (für wasserlösliche Polymere) möglich sind. Die Ölphase besteht bekanntlich meist aus mit Wasser nicht mischbaren organischen Lösungsmitteln wie aliphatischen Kohlenwasserstoffen (z.B. Hexan, Weißöl).

Beispiele für Polymerisate, die Öl-in-Wasser-Emulsionen bilden können, sind Polymerisate von Butadien, Styrol, Isopren, Chloropren, Acrylnitril, Vinylacetat, Vinyl- und Vinylidenchlorid, Alkylacrylate und -methacrylate und Copolymerisate von diesen Monomeren untereinander sowie auch mit Methylstyrol, Isobutylen oder Aethylen.

Als Beispiele für Polymerisate, die in Wasser-in-Öl-Emulsionen verwendet werden, sind die schon o.a. wasserlöslichen oder wasserquellbaren Polymerisate bzw. Copolymeriate auf Basis von (Meth-)Aaylsäurederivaten, die unvernetzt oder auch vernetzt sein können.

Das Endprodukt besteht aus den Komponenten A und B in einem Gewichtsverhältnis 20 bis 99,8 Gew%, vorzugsweise 40 bis 95 Gew%, der Komponente A, zu 0,2 bis 80 Gew%, vorzugsweise 0,5 bis 60 Gew%, der Komponente B und 0 bis 80 Gew% an neutralem Füllstoff.

Als Methode zur Bestimmung des Flüssigkeitsaufnahmevermögens wurde ein Teebeuteltest durchgeführt. Die Flüssigkeitsaufnahme von 0,2 g Prüfsubstanz wurde in einem Teebeutel nach 10 Minuten (Maximalwert) und nach dem Schleudern in einer Zentrifuge, beispielsweise in einer handelsüblichen Wäscheschleuder bei 1400 Upm, gravimetrisch ermittelt und auf 1 g Produkt umgerechnet (Retentionswert). Als Prüfflüssigkeit wurde die wäßrige 0,9%ige NaCl-Lösung verwendet.

Die Erfindung wird durch die nachfolgenden Beispiele erläutert.

### Beispiele 1 und 2:

In einem Polymerisationsgefäß wurden 423 g Acrylsäure, 625 g Acrylamid und 4,2 g N,N'-Methylenbisacrylamid in 2100 ml Wasser gelöst und mit 680 g Kalilauge (45%ig) neutralisiert. Bei Raumtemperatur wurden die Katalysatorkomponenten (1,02 g Azobisamidinpropandihydrochlorid, 0,24 g Benzyldimethylketal (Irgacure 651, Ciba Geigy AG) und 1,04 g t-Butylhydroperoxid, gelöst in Wasser) zugegeben und die adiabatische Polymerisation durch UV-Licht unter Stickstoff gestartet. Das sich bildende Polymergel wurde zerkleinert und in einer Mischeinrichtung mit handelsüblichem Düngemittel (17-17-17) als Komponente B in verschiedenen Verhältnissen vermischt. Die Zahlenfolge der Düngemittelbezeichnung bezieht sich auf den Gehalt der einzelnen Komponenten von 17 % K₂O, 17 % P₂O₅ und 17 % Stickstoff im Düngemittel.

Die Abmischung und Verarbeitung des Polymergels mit der Komponente B erfolgte in einem Mischer mit rotierenden Mischwerkzeug. Das Gerät besteht aus einem vertikal oder horizontal gelegenen Metallzylinder (Volumen ca. 6000 ml), dessen Mischwerkzeug mit Leitschaufeln, die die Wände des Mischerzylinders gründlich abstreifen, ausgerüstet ist. Nachdem eine Rührwerksdrehzahl von 300 Upm erreicht wurde, bildet sich beim Austreten der rotierenden Leitschaufeln des Mischwerkzeuges aus den heraugeschleuderten einzelnen Teilchen der Gutmasse ein Fließbett auf der ganzen Länge des Metallzylinders. In diesem Stadium erfolgt eine gleichmäßige Durchmischung und Durchknetung der beiden Komponenten.

Anschließend wurde die entstandene Polymergelmasse bei 120°C im Umlufttrockenschrank getrocknet und danach gemahlen. Das Aufnahmevermögen der Endprodukte wurde nach der Teebeuteltestmethode ermittelt (s. Tabelle 1).

**Tabelle 1:**

| | Komponente A | Komponente B | Teebeuteltest-Werte | | |
|---|---|---|---|---|---|
| | Acrylsäure/ Acrylamid-copolymerisat | Düngemittel | Maximal | Retention | |
| | Gew.% | Gew.% | (ml/g) | (a) (ml/g) | (b) (ml/g) |
| | 100 | 0 | 40,9 | 28,0 | 28,0 |
| | 0 | 100 | 0 | 0 | 0 |
| Beispiel 1: | 91 | 9 | 36,4 | 25,2 | 27,7 |
| Beispiel 2: | 83 | 17 | 33,5 | 22,9 | 27,6 |
| Bemerkung: Die Teebeuteltest-Werte Maximal und Retention (a) beziehen sich auf 1 g des zusammengesetzten Produktes aus Komponenten A und B, die Retention (b) bezieht sich auf 1 g der Komponente A im Produkt. | | | | | |

Wie aus der Tabelle zu ersehen ist, wird das Aufnahmevermögen der Zusammensetzung (Retention, Maximalwert) nur durch den Anteil an Komponente A getragen. Beim Kontakt der Zusammensetzung mit Wasser ist die Auswaschbarkeit des im gequollenen Polymerkörper eingebundenen Düngemittels deutlich langsamer als bei einer Wassereinwirkung auf die einfache Abmischung der Komponenten A und B, was bei einer praktischen Anwendung die gewünschte verzögerte Abgabe des Düngemittels an andere Körper, wie z.B. Pflanzen, ermöglicht.

### Beispiele 3 und 4:

Nach dem Verfahren gemäß Beispiel 1 wurden in einem Polymerisationsgefäß 105 g Acrylsäure, 945 g Acrylamid und 4,2 g N,N'-Methylenbisacrylamid in 2100 ml Waser gelöst, mit Kalilauge (45%ig) auf pH = 6 neutralisiert und mit einem Katalysatorsystem gemäß Beispiel 1 polymerisiert. Das sich bildende Polymergel wurde zerkleinert und in einer Mischeinrichtung mit handelsüblichem Düngemittel (17-17-17) als Komponente B in verschiedenen Verhältnissen portionsweise vermischt und bei 180°C getrocknet.

**Tabelle 2:**

| | Komponente A | Komponente B | Teebeuteltest-Werte | | |
|---|---|---|---|---|---|
| | Acrylsäure/ Acrylamidcopolymerisat | Düngemittel | Maximal | Retention | |
| | Gew.% | Gew.% | (ml/g) | (a) (ml/g) | (b) (ml/g) |
| | 100 | 0 | 25,0 | 16,6 | 16,6 |
| | 0 | 100 | 0 | 0 | 0 |
| Beispiel 3: | 91 | 9 | 22,7 | 15,2 | 16,7 |
| Beispiel 4: | 83 | 17 | 19,1 | 13,0 | 15,7 |
| Bemerkung: Die Teebeuteltestwerte Maximal und Retention (a) beziehen sich auf 1 g des zusammengesetzten Produktes aus Komponenten A und B, die Retention (b) bezieht sich auf 1 g der Komponente A im Produkt. | | | | | |

Wie aus der Tabelle zu ersehen ist, wird das Aufnahmevermögen der Zusammensetzung (Retention, Maximalwert) nur durch den Anteil an Komponente Agetragen. Beim Kontakt der Zusammensetzung mit Wasser ist die Auswaschbarkeit des im gequollenen Polymerkörper eingebundenen Düngemittels deutlich langsamer als bei einer Wassereinwirkung auf die einfache Abmischung der Komponenten A und B, was bei einer praktischen Anwendung die gewünschte verzögerte Abgabe des Düngemittels an andere Körper, wie z.B. Pflanzen, ermöglicht.

### Beispiele 5 und 6:

Nach dem Verfahren gemäß Beispiel 1 wurde ein Polymergel mit einem Vernetzergehalt von 0,3 Gew% (bezogen auf Polyacrylsäure) hergestellt und mit unterschiedlichen Mengen an Düngemittel und Cellulosekurzfasern im bereits beschriebenen Mischer vermischt und in gleicher Weise, wie in Beispielen 1 und 2 beschrieben, verarbeitet. Es entstand eine Polymermasse mit eingearbeiteten Cellulosefasern und Düngemittel, die bei 50°C getrocknet und auf ihr Aufnahmevermögen geprüft wurde. Die Ergebnisse sind in der folgenden Tabelle zusammengefaßt.

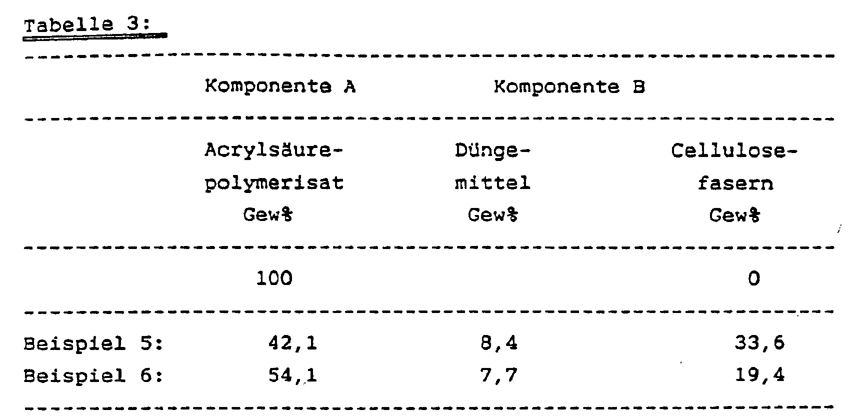

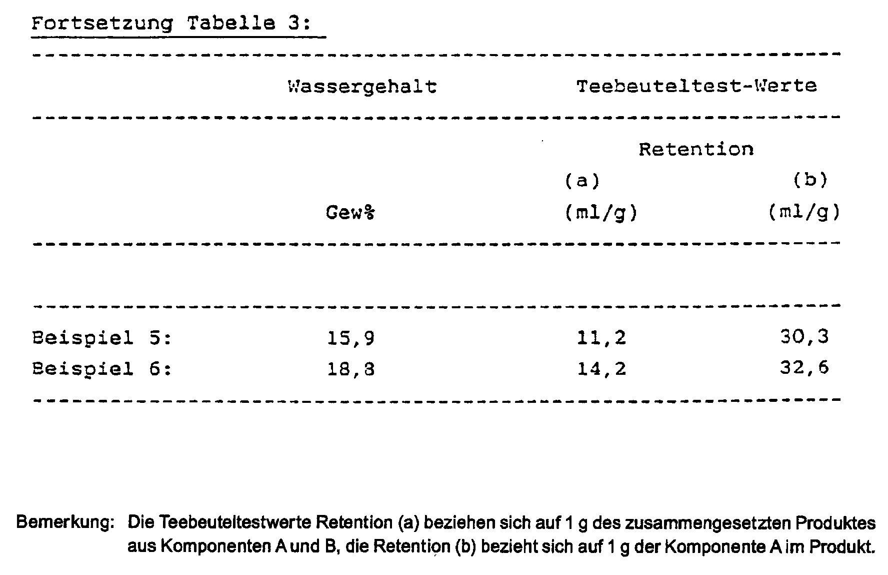

### Beispiele 7 bis 12:

In einem Polymerisationsgefäß wurden 370 g Acrylsäure, 1,1 g N,N'Methylenbisacrylamid in 410 ml Wasser gelöst und mit Natronlauge z.T. neutralisiert. Bei Raumtemperatur wurden die Katalysatorkomponenten (0,26 g Azobisamidinpropandihydrochlorid, 0,06 g Benzildimethylketal (Irgacure 651) und 0,26 g t-Butylhydroperoxid, gelöst in Wasser) zugegeben und die adiabatische Polymerisation durch UV-Licht gestartet. Das entstandene Polymergel wurde zerkleinert und in einer Mischeinrichtung gemäß den Beispielen 1 - 3 mit der Citronensäure bzw. Abmischungen der Citronensäure mit Cellulosekurzfasern vermischt und bei 120°C getrocknet. Die Zusammensetzung der Polymerprodukte in den einzelnen Beispielen und die Teebeuteltestwerte sind in nachfolgender Tabelle zusammengefaßt:

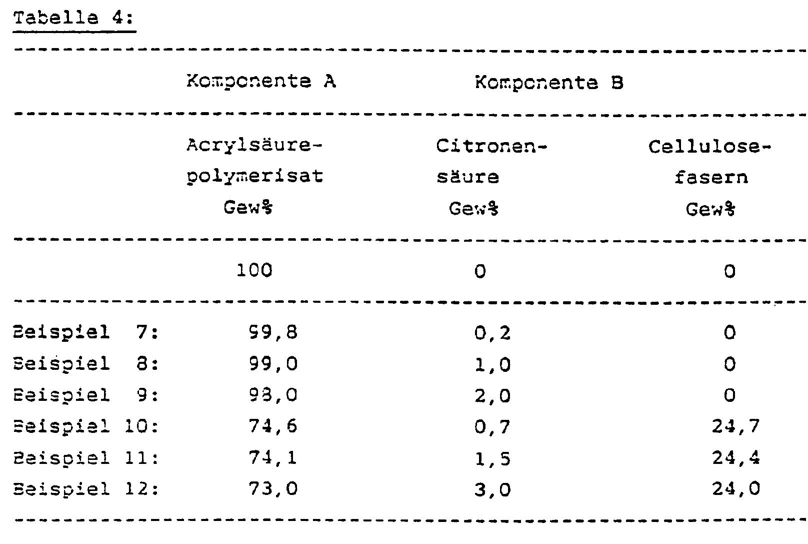

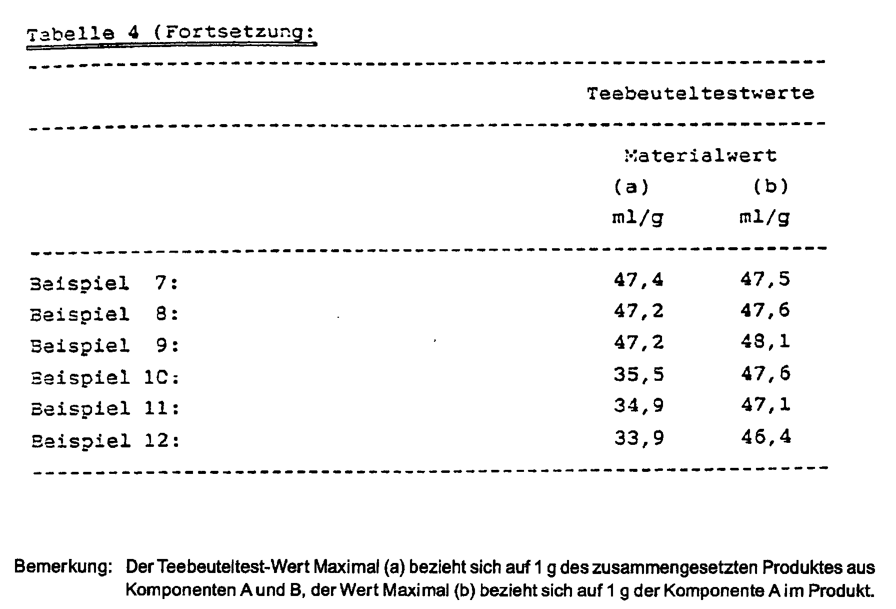

### Beispiel 13:

Eine verzögerte Abgabe des im vernetzten Polymerisat eingebauten Düngemittels wurde mit folgender Versuchsdurchführung demonstriert

Das gemäß Beispiel 1 hergestellte Polymerisat mit 16,7 Gew% Düngemittelgehalt (Einwaage 0,4 g) wurde 1:1 mit feinem Seesand (Korngröße 0,1 - 0,3 mm) vermischt und auf einer Glasfritte dreimal nacheinander jeweils mit 20 ml dest. Wasser durchgespült. Das im Gemisch nicht gebundene Wasser wurde abgesaugt und die Konzentration des Düngemittels im Eluat bestimmt.

Als Vergleichsprobe wurde 0,2 g Düngemittel ebenso mit Seesand vermischt und dreimal nacheinander mit 20 ml dest. Wasser durchgespült.

Die Ergebnisse sind in folgender Tabelle zusammengefaßt

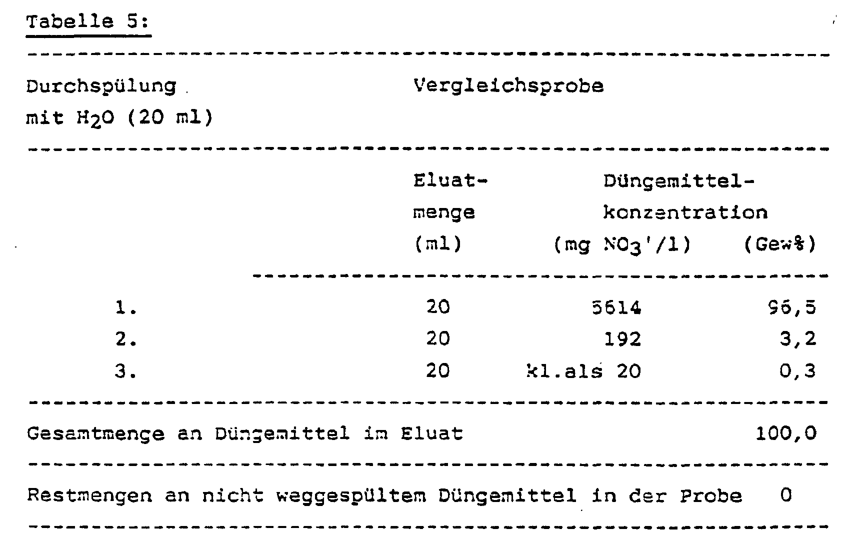

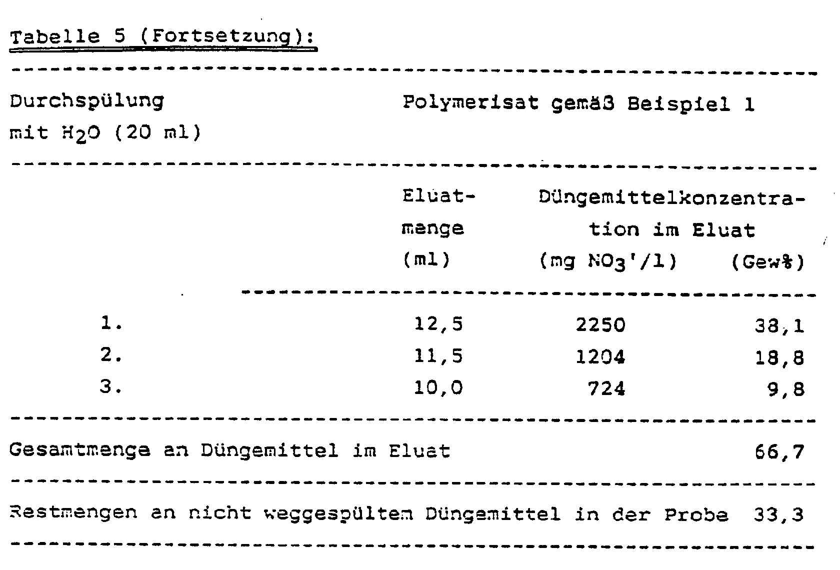

Die Ergebnisse zeigen, daß bei der Vergleichsprobe bereits nach der ersten Durchspülung mit 20 ml Wasser fast die ganze Menge des Düngemittels (96,5 Gew%) aus der Probe beseitigt wird. Im erfindungsgemäßen Polymerisat verbleiben nach dem ersten Spülgang immer noch 62 Gew% Düngemittel im gequollenen Polymergel gebunden und nach drei Spülgängen sogar noch 33,3 Gew%, indem in der Vergleichsprobe das Düngemittel nach drei Spülgängen vollständig ausgewaschen ist.

### Beispiel 14:

In diesem Beispiel wurde eine verzögerte Abgabe des im vernetzten Polymerisat enthaltenen Düngemittels demonstriert, wobei der Düngemittelgehalt in der Sandprobe und die Wassermengen zur Berieselung so gewählt wurden, daß sie den praxisnahen Bedingungen entsprechen.
1. Als Vergleichsbeispiel zur Ermittlung der Auswasch bar keit eines Düngemittels aus der Bodenprobe (ohne Superabsorberzusatz) wurde zunächst ein handelsübicher Kunstdünger 17-17-17 mit feinem Seesand (Korngröße 0,1 - 0,3 mm) vermischt, auf einer Glasfritte (Durchmesser 90 mm) mehrmals mit Wasser berieselt und im Filtrat die Konzentration des ausgewaschenen Düngemittels ermittelt.
   Die Berieselung der Sandschicht erfolgte über eine gelochte Platte (Gießkanneneffekt), und die Wassermenge wurde so gewählt, daß sie in etwa der Wassermenge bei einem Starkregen (5-8 l/m² innerhalb von 5 min.) entsprach. Die Sandschicht von 120 g Seesand mit Zusatz von 0,12 Kunstdünger (0,1 Gew%) war ca. 1 cm hoch und benötigte 47,5 g Wasser zur vollständigen Sättigung mit Wasser. Für die erste Berieselung wurden daher insgesamt 100 ml Wasser verwendet, so daß nach der Sättigung der Sandschicht mit Wasser ca. 50 ml als Filtrat durch die Sandschicht durchgeflossen sind. Die nächste und weitere Berieselungen wurden mit 50 ml Wasser durchgeführt. Nach jeder Berieselung wurde im Filtrat die Düngemittelmenge ermittelt Nach dreimaligem Berieseln mit Wasser wurde die feuchte Sandschicht noch zweimal mit der Petrilösung (0,118%ige Kunstdünger-17-17-17-Lösung) berieselt und die Düngemittelmenge im Filtrat ebenfalls ermittelt Die Ergebnisse sind in den folgenden Tabellen 6 und 7 zusammengefaßt. 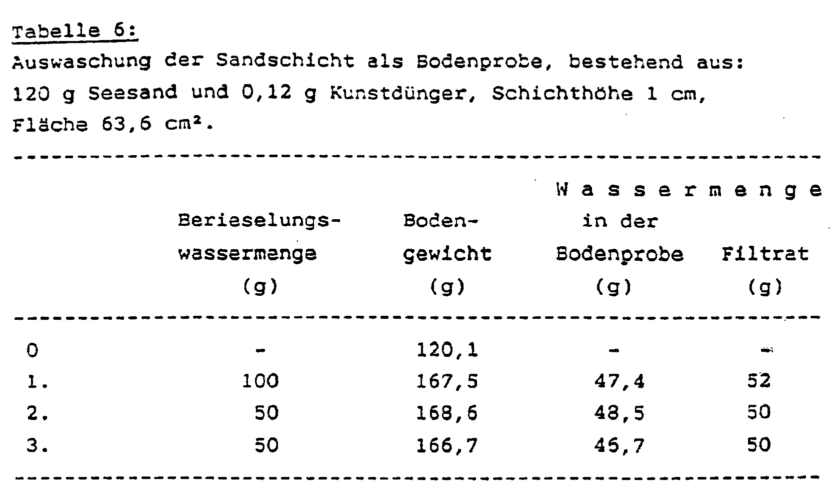 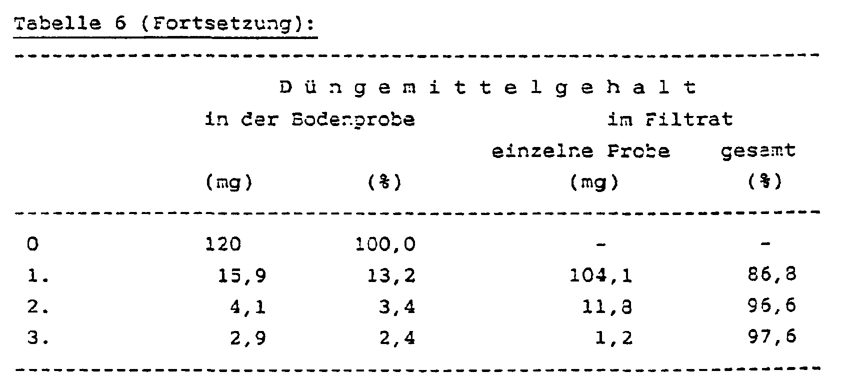 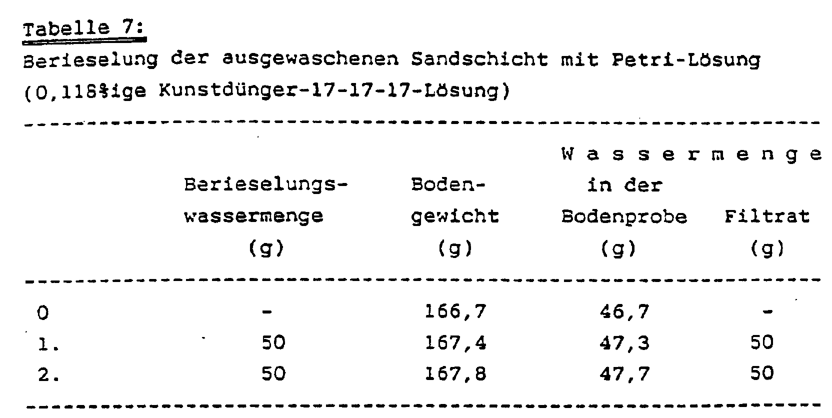 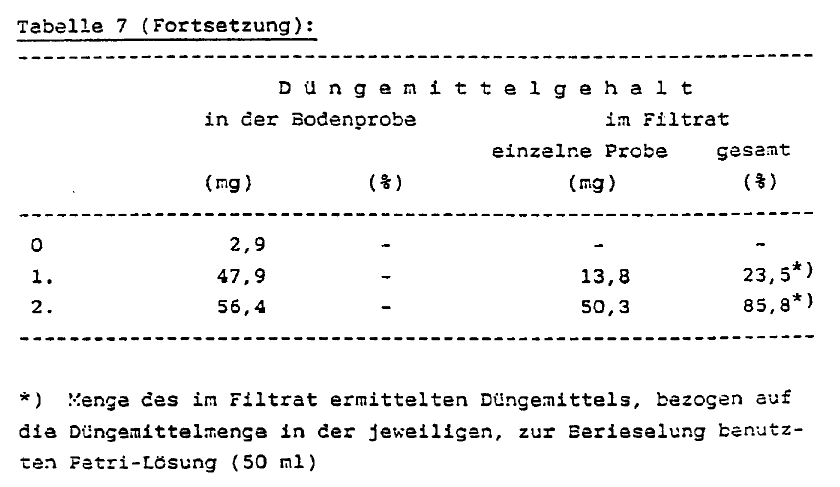
2. Zur Ermittlung derAuswaschbarkeit eines Düngemittels aus einer Bodenprobe mit Superabsorberzuatz wurden zunächst 20 Gew% Kunstdünger 17-17-17 in das Polymergel gemäß Beispiel 1 eingearbeitet, das Polymergel getrocknet und gemahlen. Die getrocknete düngemittelhaltige Probe (0,6 g) wurde dann mit 240 g feinem Seesand vermischt und wie im vorigen Fall mit Wasser mehrmals berieselt, wobei nach jeder Berieselung die ausgewaschene Düngemittelmenge im Filtrat ermittelt wurde. Nach fünfmaliger Berieselung wurde anschließend die feuchte Sandschicht noch zweimal mit Petri-Lösung durchgespült und der Düngemittelgehalt in der superabsorberhaltigen Sandschicht ermittelt Die Ergebnisse sind in den Tabellen 8 und 9 zusammengefaßt.

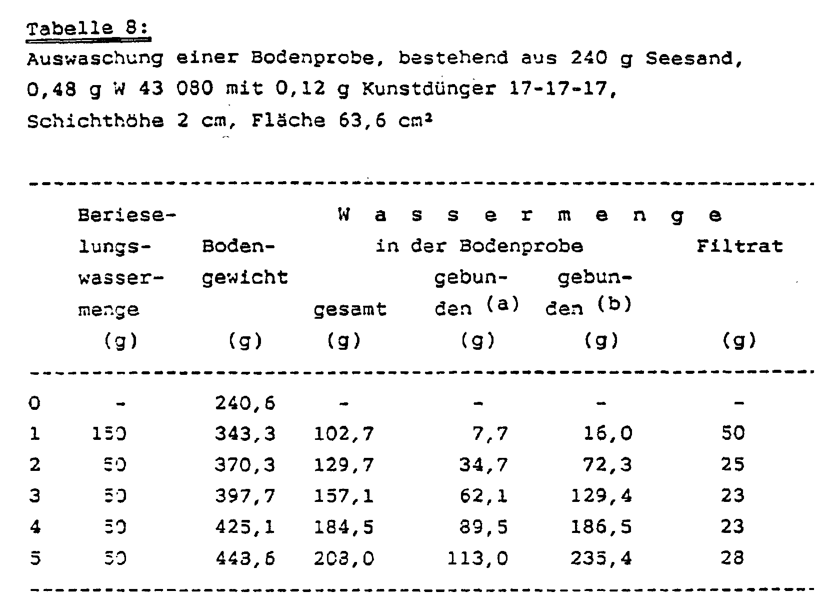

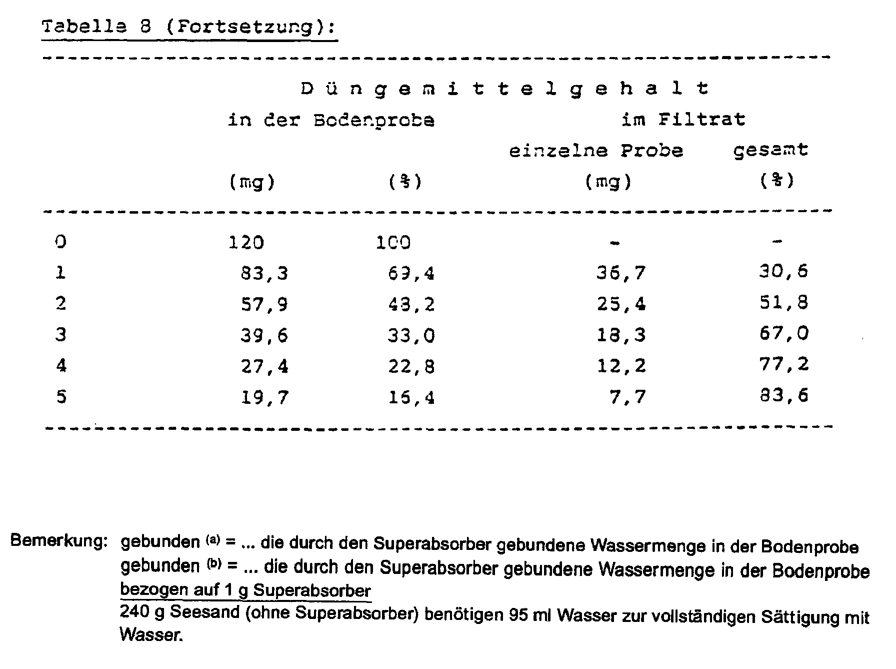

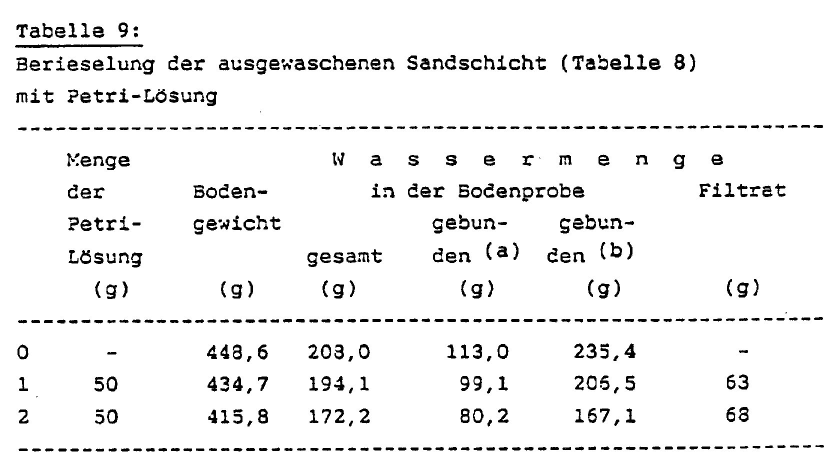

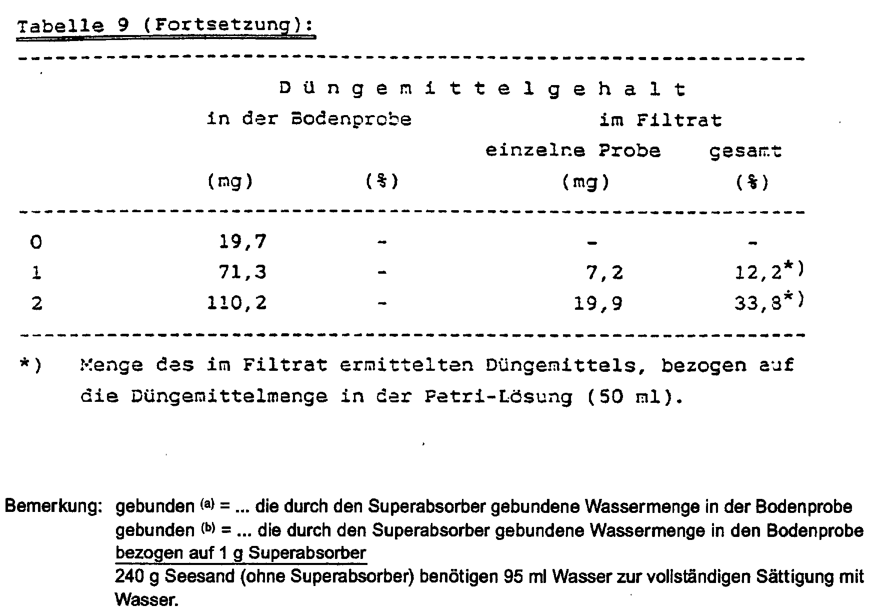

Wenn man jetzt die Tabellen 6 und 8 vergleicht, sieht man, daß die Auswaschung des Düngemittels aus der Sandschicht ohne Superabsorberzusatz bereits nach einmaliger Berieselung (Tabelle 6) zu 57 % erfolgte und nach zweimaliger Berieselung praktisch vollständig war (d.h. bis zu 97 %). Dagegen erfolgte bei einem Zusatz von düngemittelhaltigem Superabsorber zu gleicher Sandprobe die Auswaschung des Düngemittels aus der Sandschicht deutlich langsamer (s. Tabelle 8). Nach erster Berieselung der mit Wasser vollgesättigten Bodenprobe blieben noch 70 % und nach zweiter Berieselung immer noch 45 % des Düngemittels in der Sandschicht.

Auch bei der Berieselung der vom Düngemittel ausgewaschenen Sandschicht mit Petri-Lösung (s. Tabellen 7 und 8) zeigte sich, daß mehr Düngemittel aus der Petri-Lösung in der Sandschicht gebunden blieb, in der das synthetische, vernetzte Polymerisat vorhanden war (Tabelle 9).

Die zur Berieselung benutzte Wassermenge von 50 ml entspricht auf der Prüffläche von 63,6 cm² einem Niederschlag von 7,86 mm. Die Gesamtmenge von Wasser (350 ml), die in der Tabelle 8 zur Berieselung benutzt wurde, entspricht daher einem in relativ kurzer Zeit gefallenen Niederschlag von 55,0 mm, was wiederum mit einem tropischen Starkregen (35 - 50 mm Niederschlag in 2 bis 3 Stunden) vergleichbar wäre. Bei einem Starkregen in unseren klimatischen Bedingungen sind das nur 5 - 8 mm Niederschlag.

Die Auswaschbarkeit des Düngemittels wurde in einer 2 cm dicken Schicht ermittelt, die mehrmals mit Wasser berieselt wurde. Durch diese relativ dünne Schicht konnte das Wasser schnell durchlaufen, so daß die Quellung des Superabsorbers, die zeitabhängig ist, nur unvollständig verlaufen konnte, wie man dies auch in Tabelle 8 (gebundene Wassermenge in der Bodenprobe) nach jeder jeweiligen Berieselung verfolgen kann. Trotzdem nimmt die gebundene Wassermenge in der Bodenprobe nach jeder Berieselung deutlich zu. Diese 2 cm dicke Schicht stellt die oberste Schicht der Erdprobe dar, die eigentlich vom Regen am meisten durchgespült wird. In einem den wirklichen Bedingungen bei der Pflanzenzüchtung entsprechenden Versuch mit einer Bodenschichtdicke von mehr als 2 cm wird der Wasserdurchlaufdurch diese dickere Bodenschicht langsamer, so daß noch mehr Wasser vom Superabsorber aufgenommen werden kann und dadurch auch weniger Düngemittel aus der Bodenschicht ausgewaschen wird.

## Patentansprüche

1. Verfahren zur Herstellung von Absorptionsmaterial für Wasser, wäßrige Lösungen und Körperflüssigkeiten, bestehend aus wenigstens zwei Komponenten A und B, wobei die Komponente A wenigstens ein wasserquellbares synthetisches Polymeres oder Copolymeres und die Komponente B, mit Ausnahme von Tensiden eines HLB-Wertes von mindestens 3, Homopolymeren des Ethylenoxids, Blockcopolymere von Ethylenoxid und Alkylenoxiden sowie deren polyfunktionellen Blockcopolymeren, wenigstens eine natürliche oder synthetische Verbindung ist, die bei normaler Temperatur als rieselfähiges Pulver, das in Wasser gut oder wenigstens begrenzt löslich ist, oder als eine Flüssigkeit vorliegt,
**dadurch gekennzeichnet, daß**
die Komponente B als Pulver oder in flüssiger Form oder als Lösung der Komponente A in der Endphase ihres Herstellungsprozesses d.h. in der Endphase der Polymerisation nach Erreichung eines Polymerumsatzes von mindestens 90 %, bevorzugt mindestens 95 %, zugesetzt, mit dem Polymergel der Komponente A gleichmäßig durchmischt und ggf. zwecks Erhaltung eines pulverförmigen, rieselfähigen Endprodukts getrocknet und gemahlen wird.

2. Verfahren nach Anspruch 1, **dadurch gekennzeichnet, daß** die Komponente B in der Endphase der Polymerisation nach Erreichen eines Polymerumsatzes von **mehr als** 98 % zu der Komponente A zugesetzt wird.

3. Verfahren nach Anspruch 1 oder 2, **dadurch gekennzeichnet, daß** die Komponente B in getrocknetem Zustand als Pulver oder in wäßriger Lösung verwendet wird.

4. Verfahren nach einem der Ansprüche 1 bis 3, **dadurch gekennzeichnet, daß** die Komponente A ein vernetztes Polymeres oder Copolymeres auf der Basis von Acrylsäure, Methacrylsäure, Derivaten dieser Carbonsäuren, vorzugsweise ein Homo- oder Copolymerisat der Acryl-, Methacryl-, 2-Acrylamido-2-methylpropansulfonsäure, den Alkali- oder Ammoniumsalzen dieser Carbonsäuren, des Acryl- oder Methacrylamids oder deren Derivaten, des Vinylpyrrolidons sowie deren Copolymerisaten untereinander oder mit anderen nur teilweise wasserlöslichen Monomeren, vorzugsweise Vinylacetat, ist.

5. Verfahren nach einem der Ansprüche 1 bis 4, **dadurch gekennzeichnet, daß** die Komponente A ein vernetztes Polymeres oder Copolymeres ist, wobei als Vernetzer di- und mehrfunktionelle Verbindungen verwendet werden.

6. Verfahren nach einem der Ansprüche 1 bis 5, **dadurch gekennzeichnet, daß** als Komponente B ein Düngemittel verwendet wird.

7. Verfahren nach einem der Ansprüche 1 bis 6, **dadurch gekennzeichnet, daß** zusätzlich zur Komponente B noch wenigstens ein Polysaccharid oder Polysaccharidderivat, bevorzugt modifizierte Cellulose, Stärke, Dextrin oder Gummiharz bzw. ihre Abmischung zugesetzt wird.

8. Verfahren nach einem der Ansprüche 1 bis 6, **dadurch gekennzeichnet, daß** zusätzlich zur Komponente B Naturfasern, vorzugsweise Wolle, Seide, Baumwolle; oder Cellulosefasern, bevorzugt Viskose-, Acetat- oder Triacetatfasern; oder synthetische Fasern, bevorzugt Polyester-, Polyolefin-, Polyamid-, Polyvinylalkohol-, Polyurethan-, Polyharnstoff- oder Polyacrylnitrilfasern, bevorzugt in Kurzfaserschnittform, zugesetzt werden.

9. Verfahren nach einem der Ansprüche 1 bis 6, **dadurch gekennzeichnet, daß** zu der Komponente A ein neutraler Füllstoff im Gemisch mit der Komponente B, bevorzugt Sand, Torf, Tonerde, Erde für die Pflanzenzucht, andere Düngemittel, Farbstoffe, Pigmente, gemahlene Nußschalen oder Kernobstschalen, Holzmehl, chitinhaltiges Mehl oder die gemahlene Komponente A selbst in Pulver- oder teilgequollener Form eingearbeitet wird.

10. Verfahren nach einem der Ansprüche 1 bis 9, **dadurch gekennzeichnet, daß** das Endprodukt 20 bis 99,8 Gew.-%, bevorzugt 40 bis 95 Gew.-% an Komponente A und 0,2 bis 80 Gew.-%, vorzugsweise 0,5 bis 30 Gew.-% an Komponente B und 0 bis 80 Gew.-% an neutralem Füllstoff enthält.

11. Absorptionsmaterial erhältlich nach Anspruch 8, **dadurch gekennzeichnet, daß** die durch Diffusion kontrollierte Beweglichkeit der Komponente B in dem mit Wasser gequollenen Absorptionsmaterial niedriger ist als in einem physikalischen Gemisch der beiden Komponenten A und B und das Absorptionsmaterial zusätzlich zur Komponente B Naturfasern, bevorzugt Wolle, Seide, Baumwolle; oder Cellulosefasern, bevorzugt Viskose-, Acetatoder Triacetatfasern; oder synthetische Fasern, bevorzugt Polyester-, Polyolefin-, Polyamid-, Polyvinylalkohol,- Polyurethan-, Polyharnstoff- oder Polyacrylnitrilfasern, bevorzugt in Kurzfaserschnittform, enthält.

12. Verwendung eines Absorptionsmaterials nach Anspruch 11 zur Aufnahme und/oder Zurückhaltung von Wasser und/oder wäßrigen Lösungen und zur nachfolgenden gesteuerten Abgabe von Wasser und der im aufgequollenen Polymergel enthaltenen und in wäßrigem Medium löslichen Substanzen (Komponente B) an andere Körper, vorzugsweise Pflanzen, als Nährboden für verschiedene Kulturen und beim gesteuerten Dosieren von Nährstoffen oder Arzneimitteln.

13. Verwendung des nach einem der Ansprüche 1 bis 10 erhaltenen Absorptionsmaterials zur Aufnahme und/oder Zurückhaltung von Wasser und/oder wäßrigen Lösungen und zur nachfolgenden gesteuerten Abgabe von Wasser und der im aufgequollenen Polymergel enthaltenen und in waßrigem Medium löslichen Substanzen (Komponente B) an andere Körper mit Ausnahme von Düngemittel, vorzugsweise Pflanzen, beim gesteuerten Dosieren von Nährstoffen oder Arzneimitteln.

## Claims

1. A process for the production of absorbing material for water, aqueous solutions and body liquids, the absorbing material consisting of at least two components A and B, wherein component A is at least a water swellable synthetic polymer or copolymer, and component B is, with the exception of surfactants having a HLB value of at least 3, homopolymers of ethylene oxide, block copolymers of ethylene oxide and alcohol ethers as well as their multifunctional block copolymers, at least a natural or synthetic compound which, at normal temperature, is present as pourable powder highly or at least partially soluble in water or as a liquid, **characterized in that** component B is added to component A in the form of a powder or liquid or as solution during the end phase of the manufacturing process of component A, that is to say in the end phase of the polymerization, after a polymer conversion of at least 90 %, preferably at least 95 % has been attained, that is mixed uniformly with the polymer gel of component A and, for the purpose of obtaining a powdery, pourable end product, is dried, if necessary, and ground.

2. The process according to claim 1, **characterized in that** component B is added to component A in the end phase of the polymerization, after a polymer conversion of more than 98 % has been attained.

3. The process according to claim 1 or 2, **characterized in that** component B is used in dried state as a powder or in an aqueous solution.

4. The process according to any one of claims 1 to 3 **characterized in that** component A is a cross-linked polymer or copolymer based on acrylic acid, methacrylic acid, derivatives of said carboxylic acids, preferably is a homo- or copolymer of acrylic acid, methacrylic acid, 2-acrylamido-2-methylpropane sulfonic acid, of the alkali- or ammonium salts of said carboxylic acids, of acrylamide or methacrylamide and their derivatives, of vinyl pyrrolidone as well as the copolymers thereof with one another or with other, only partially water-soluble monomers, preferably vinyl acetate.

5. The process according to any one of claims 1 to 4 **characterized in that** component A is a cross-linked polymer or copolymer, with difunctional and polyfunctional compounds being used as cross-linking agents.

6. The process according to any one of claims 1 to 5, **characterized in that** a fertilizer is used as component B.

7. The process according to any one of claims 1 to 6 **characterized in that** in addition to compound B at least one polysaccharide or polysaccharide derivative, preferably modified cellulose, starch, dextrin or gum resin or a mixture thereof is added.

8. The process according to any one of claims 1 to 6 **characterized in that** in addition to component B natural fibers, preferably wool, silk, cotton, or cellulose fibers, preferably fibers of viscose, acetate, or triacetate, or synthetic fibers, preferably fibers of polyester, polyolefin, polyamide, polyvinyl alcohol, polyurethane, poly-urea, or polyacrylonitrile, preferably in the form of short fibers, are added.

9. The process according to any one of claims 1 to 6 **characterized in that** a neutral filling agent, preferably sand, peat, clay, garden mold for the cultivation of plants, other fertilizers, dyestuff, pigments, ground shells of nuts or pomaceous fruit, wood flour, chitin-containing flour, in admixture with component B, or the ground component A itself in powder or partially swollen form is incorporated into component A.

10. The process according to any one of claims 1 to 9 **characterized in that** the end product comprises 20 to 99,8 %-wt, preferably 40 to 95 %-wt of component A, and 0,2 to 80 %-wt., preferably 0,5 to 30 %-wt. of component B and 0 to 80 %-wt. of neutral filler.

11. An absorbing material obtainable according to claim 8 **characterized in that** the diffusion-controlled movability of component B is lower in the water-swollen absorbing material than in a physical mixture of the two components A and B and the absorbing material contains in addition to component B natural fibers, preferably wool, silk, cotton, or cellulose fibers, preferably fibers of viscose, acetate or triacetate, or synthetic fibers, preferably fibers of polyester, polyolefin, polyamide, polyamide, polyvinyl alcohol, polyurethane, poly-urea, or polyacrylonitrile, preferably in the form of short fibers.

12. The use of an absorbing material according to claim 11 for the absorption and/or retention of water and/or aqueous solutions and for the subsequent controlled release of water and of substances (component B) comprised in the swollen polymer gel and soluble in the aqueous medium to other bodies, preferably plants, as nutrients for various cultures, and in the controlled dosage of nutrients or drugs.

13. The use of the absorbing material obtained by one of claims 1 to 10 for the absorption and/or retention of water and/or aqueous solutions and for the subsequent controlled release of water and of the substances (component B) comprised in the swollen polymer gel and soluble in the aqueous medium, with the exception of fertilizer, to other bodies, preferably plants, and in the controlled dosage of nutrients or drugs.

## Revendications

1. Procédé de préparation d'une matière d'absorption pour l'eau, des solutions aqueuses et des liquides corporels, qui se compose d'au moins deux composants A et B, où le composant A est au moins un polymère ou copolymère, gonflable à l'eau et le composant B est, à l'exception de surfactants d'une valeur HLB d'au moins 3, d'homopolymères d'oxyde d'éthylène, de copolymères bloc d'oxyde d'éthylène et d'oxyde d'alkylène ainsi que de leurs copolymères bloc polyfonctionnels, au moins un composé naturel ou de synthèse, qui se présente à la température normale, sous forme d'une poudre fluide, qui est bien soluble dans l'eau ou qui y est soluble de manière au moins limitée, ou qui se présente sous forme d'un liquide, **caractérisé en ce que** l'on ajoute le composant B sous forme de poudre, sous forme liquide ou sous forme de solution, au composant A, dans la phase finale de son processus de fabrication, c'est à dire dans la phase finale de la polymérisation, après avoir atteint une conversion en polymère d'au moins 90 %, de préférence d'au moins 95 %, on le mélange uniformément au gel polymérique du composant A et on le sèche et broie éventuellement dans le but d'obtenir un produit final pulvérulent, fluide.

2. Procédé suivant la revendication 1, **caractérisé en ce que** l'on ajoute le composant B au composant A au cours de la phase finale de la polymérisation, après avoir atteint une conversion de polymère supérieure à 98 %.

3. Procédé suivant la revendication 1 ou 2, **caractérisé en ce que** l'on utilise le composant B à l'état séché, sous forme de poudre, ou en solution aqueuse.

4. Procédé suivant l'une quelconque des revendications 1 à 3, **caractérisé en ce que** le composant A est un polymère ou copolymère réticulé, à base d'acide acrylique, d'acide méthacrylique, de dérivés de ces acides carboxyliques, de préférence un homopolymère ou copolymère de l'acide acrylique, méthacrylique, 2-acrylamido-2-méthyl-propane-sulfonique, des sels de métaux alcalins ou d'ammonium de ces acides carboxyliques, de l'acrylamide ou du méthacrylamide et de leurs dérivés, de la vinylpyrrolidone, comme aussi de leurs copolymères les uns avec les autres ou avec d'autres monomères seulement partiellement solubles dans l'eau, de préférence l'acétate de vinyle.

5. Procédé suivant l'une quelconque des revendications 1 à 4, **caractérisé en ce que** le composant A est un polymère ou copolymère réticulé, où l'on utilise à titre d'agents de réticulation, des composés di- et polyfonctionnels.

6. Procédé suivant l'une quelconque des revendications 1 à 5, **caractérisé en ce qu'**à titre de composant B on utilise un engrais.

7. Procédé suivant l'une quelconque des revendications 1 à 6, **caractérisé en ce que** complémentairement au composant B, on ajoute au moins encore un polysaccharide ou un dérivé de polysaccharide, de préférence de la cellulose modifiée, de la fécule, de la dextrine ou de la résine de gomme, ou leurs mélanges.

8. Procédé suivant l'une quelconque des revendications 1 à 6, **caractérisé en ce que** complémentairement au composant B, on ajoute des fibres naturelles, de préférence de la laine, de la soie, du coton, ou des fibres de cellulose, de préférence des fibres de viscose, d'acétate, ou de triacétate, ou des fibres synthétiques, de préférence des fibres de polyester, de polyoléfine, de polyamide, de poly(alcool vinylique), de polyuréthanne, de polyurée ou de polyacrylonitrile, de préférence sous forme coupée en fibres courtes.

9. Procédé suivant l'une quelconque des revendications 1 à 6, **caractérisé en ce que** l'on incorpore au composant A une charge neutre en mélange au composant B, de préférence du sable, de la tourbe, de l'alumine, du terreau pour la culture des plantes, d'autres engrais, des colorants, des pigments, des écales de noix broyées ou des endocarpes de drupes broyés, de la sciure de bois, de la farine contenant de la chitine ou le composant A broyé lui-même sous forme de poudre ou partiellement gonflé.

10. Procédé suivant l'une quelconque des revendications 1 à 9, **caractérisé en ce que** le produit final contient 20 à 99,8 % en poids, de préférence 40 à 95 % en poids, de composant A et de 0,2 à 80 % en poids, de préférence de 0,5 à 30 % en poids, de composant B et de 0 à 80 % d'une charge neutre.

11. Matière d'absorption que l'on peut obtenir suivant la revendication 8, **caractérisée en ce que** la mobilité du composant B, réglée par diffusion, dans la matière d'absorption gonflée à l'eau est plus faible que dans un mélange physique des deux composants A et B et **en ce que** la matière d'absorption complémentairement au composant B contient des fibres naturelles, de préférence de la laine, de la soie, du coton, ou des fibres de cellulose, de préférence des fibres de viscose, d'acétate, ou de triacétate, ou des fibres synthétiques, de préférence des fibres de polyester, de polyoléfine, de polyamide, de poly(alcool vinylique), de polyuréthanne, de polyurée ou de polyacrylonitrile, de préférence sous forme coupée en fibres courtes.

12. Utilisation d'une matière d'absorption suivant la revendication 11 pour la prise et/ou la rétention d'eau et/ou de solutions aqueuses et pour la libération réglée subséquente d'eau et des substances (composant B) contenues dans le gel polymérique gonflé et solubles en milieu aqueux à d'autres corps, de préférence des plantes, à titre de couches nourricières pour diverses cultures et au cours du dosage réglé de substances nutritives ou de médicaments.

13. Utilisation de la matière d'absorption obtenue suivant l'une des revendications 1 à 10 pour la prise et/ou la rétention d'eau et/ou de solutions aqueuses et pour la libération réglée subséquente d'eau et des substances (composant B) contenues dans le gel polymérique gonflé et solubles en milieu aqueux, à l'exception d'engrais, à d'autres corps, de préférence des plantes, et au cours du dosage réglé de substances nutritives ou de médicaments.
